# EUROPEAN PATENT APPLICATION

(11) **EP 4 151 225 A1**
(43) Date of publication of application: **22.03.2023**
(21) Application number: 21870878.2
(22) Date of filing: 18.06.2021
(51) Int. Cl.: A61K 35/74, C12N 1/20, C12N 1/04, A61K 9/48, C12Q 1/04, A61P 1/00, C12R 1/01

(54) **WHOLE BACTERIA CAPSULE, PREPARATION METHOD THEREFOR AND USE THEREOF**

(30) Priority: 22.09.2020 CN 202011002001
(71) Applicant: Suzhou Precision Biotech Co., Ltd, Suzhou, Jiangsu 215123 (CN)
(72) Inventor: LU, Min, Suzhou, Jiangsu 215123 (CN); BU, Wenli, Suzhou, Jiangsu 215123 (CN); LIU, Dan, Suzhou, Jiangsu 215123 (CN); ZHANG, Shuilong, Suzhou, Jiangsu 215123 (CN); JIN, Hongmei, Suzhou, Jiangsu 215123 (CN); ZHU, Yongliang, Suzhou, Jiangsu 215123 (CN); ZHU, Mengmeng, Suzhou, Jiangsu 215123 (CN); MU, Xiaojing, Suzhou, Jiangsu 215123 (CN)
(74) Representative: Nony
(86) International application number: PCT/CN2021/100784
(87) International publication number: WO 2022/062496

(57) **Abstract**

Provided are a whole bacteria capsule, a preparation method therefor and a use thereof. The preparation method comprises the steps of: (1) screening fecal bacteria donors using high-throughput sequencing; (2) collecting feces of the fecal bacteria donors screened in step (1), and preparing a fecal bacterial liquid; and (3) mixing the fecal bacterial liquid prepared in step (2) with a freeze-drying protective agent, cooling, freezing and vacuum-drying the mixture, and loading the freeze-dried feces bacteria powder obtained into a capsule shell to obtain the whole bacteria capsule. By screening the fecal bacteria donors using high-throughput sequencing, fecal bacteria donors can be quickly screened for different fecal bacteria receptors, and the whole bacteria capsule prepared can accurately match with the fecal bacteria receptors.

## Description

### TECHNICAL FIELD

The present application belongs to the technical field of fecal microbiota transplantation and relates to a whole bacteria capsule, a preparation method thereof and a use thereof.

### BACKGROUND

Fecal microbiota transplantation (FMT) is a technology for transplanting a functional flora from healthy human feces into an intestinal tract of a patient, establishing a new intestinal flora in the intestinal tract of the patient and treating intraintestinal and extraintestinal diseases. In the recent ten years, the FMT has made breakthroughs in the treatment of intraintestinal and extraintestinal diseases, and great hopes are clinically placed on the FMT treatment of difficult intraintestinal and extraintestinal diseases. However, due to the complex establishment of an FMT methodology and the lack of a uniform standard at home and abroad, various types of researches have relatively large heterogeneity in therapeutic effect, significantly limiting a clinical popularization and application of the FMT. At present, fecal microbiota transplantation has a good effect in treating diseases caused by intestinal flora imbalance such as *Clostridium difficile* infection, irritable bowel syndrome and inflammatory bowel disease. Clinically, the FMT is mainly performed through enema, nasogastric tube, duodenum, jejunum and colonoscope, but these methods all relate to invasive transplantation, resulting in poor patient experience.

CN209967402U discloses a transgastroenteroscopic fecal microbiota transplantation bolus catheter, which includes a hollow handle and a catheter for the bolus of fecal bacteria. A connection portion for connecting the catheter is disposed at one end of the handle, and an injection tube for injecting the fecal bacteria is disposed on a side of the handle facing away from the catheter. The catheter is used for fecal microbiota transplantation so that a whole injection process can be performed under a visual condition and the fecal bacteria can be avoided contaminating an inspection instrument, enhancing the standardization of operation under an endoscope of the fecal microbiota transplantation. However, this apparatus requires to be used in conjunction with the enema treatment, which is prone to cause a secondary injury to a patient with a relatively weak constitution or intestinal erosion damage.

CN110638783A discloses a method for preparing a capsule. Healthy and high-quality feces of a fecal microbiota transplantation donor are used for simulating enzymatic hydrolysis of radix codonopsis saponin by intestinal microorganisms in vitro, and through a suitable fermentation condition, radix codonopsis saponin is converted into active small-molecule substances of secondary saponins, exerting a pharmaceutical effect. A fermentation broth of fecal bacteria containing the active small-molecule substances is loaded into an enteric capsule shell for administration to a patient so that the patient can better absorb the active substances while an intestinal flora is improved, which is beneficial to health. However, this solution does not perform operations such as preservation on beneficial bacteria, and the beneficial bacteria are prone to inactivation. Therefore, the capsule requires to be used as soon as possible in a short time and is not suitable for long-term preservation.

CN109010380A discloses a process for an intestinal flora preparation in the clinical treatment of fecal microbiota transplantation. The process includes three steps: collection of feces, preparation of the intestinal flora preparation and filling the intestinal flora preparation, where the preparation of the intestinal flora preparation uses a new separation method of "aerobic+anaerobic" which maximizes the protection of the flora diversity of "aerobic bacteria" and "anaerobic bacteria" in an intestinal tract, and a glycerol protective agent is added to maintain the activity of bacteria. However, in this process, a certain volume of suspension requires to be mixed with a glycerol solution during operation, and the prepared intestinal flora preparation has a low concentration of flora and a low pharmaceutical effect.

At present, the fecal microbiota transplantation is mainly performed through a mechanical intervention method with a complex process, and the patient has a certain pain. How to provide a fast and efficient new FMT technology, which can not only relieve the pain of patient, but also provide a sufficient number of beneficial bacteria for disease treatment or symptom improvement, has become an urgent problem to be solved.

### SUMMARY

The present application provides a whole bacteria capsule, a preparation method thereof and a use thereof. The whole bacteria capsule is loaded with lyophilized powder of flora of a fecal bacteria donor screened through high-throughput sequencing, improves the efficiency and comfort of fecal microbiota transplantation and has a wide application prospect clinically.

In a first aspect, the present application provides a preparation method of a whole bacteria capsule. The preparation method includes the following steps:
(1) screening a fecal bacteria donor through high-throughput sequencing;
(2) collecting feces of the fecal bacteria donor screened in step (1) to prepare a fecal bacteria liquid; and
(3) mixing the fecal bacteria liquid prepared in step (2) with a lyophilizing protective agent and cooling, freezing and vacuum drying the mixture to obtain lyophilized powder of fecal bacteria which is loaded into a capsule shell to obtain the whole bacteria capsule.

In the present application, the fecal bacteria donor is screened through the high-throughput sequencing, which is conducive to accurately identifying a flora composition of fecal bacteria donor and has advantages in discovering unknown species and pathogens of fecal bacteria donor. A detection resolution reaches a strain level, achieving an effect of detecting multiple fecal bacteria donors at one time.

Preferably, a method for screening the fecal bacteria donor through the high-throughput sequencing in step (1) includes the following steps:
(1') performing second-generation sequencing on DNA and/or RNA extracted from the fecal bacteria donor to obtain original sequencing data;
(2') after removing a host gene from the original sequencing data, comparing the sequencing data with a microbial database for bacterial strains identification and abundance detection;
(3') comparing the sequencing data with a pathogenic bacteria database to confirm no pathogenic bacteria in the fecal bacteria donor; and
(4') comparing the sequencing data with an intestinal flora of a fecal bacteria receptor and screening to obtain a fecal bacteria donor complementary to the fecal bacteria receptor.

In the present application, the fecal bacteria composition of the fecal bacteria donor is identified through the high-throughput sequencing and compared with the intestinal flora of the fecal bacteria receptor (the patient), which is conducive to screening different fecal bacteria donors for different fecal bacteria receptors, achieving an "accurate match" and improving a pharmaceutical effect. Moreover, the high-throughput sequencing can also identify pathogenic bacteria, which is conducive to excluding a fecal bacteria donor with a potential risk and performing quality monitoring.

Preferably, the microbial database in step (2') includes any one or a combination of at least two of a bacterial genome, a fungal genome or a viral genome derived from a public database.

Preferably, the pathogenic bacteria database in step (3') includes a pathogenic bacterial genome derived from the public database.

In the present application, public databases such as National Center for Biotechnology Information (NCBI) and Kyoto Encyclopedia of Genes and Genomes (KEGG) are used for constructing the microbial database and the pathogenic bacteria database, and the sequencing data of the fecal bacteria donor is compared with the above databases so that microorganism identification at the strain level is achieved and a fecal bacteria donor carrying the pathogenic bacteria is excluded, reducing a health risk.

Preferably, screening the fecal bacteria donor through the high-throughput sequencing in step (1) is performed according to a biomarker expressed by the fecal bacteria donor and a diversity index of the biomarker.

Preferably, the biomarker includes any one or a combination of at least two of *Escherichia coli, Clostridium ramosum, Eubacterium cylindroides, Roseburia hominis, Faecalibacterium prausnitzii, Bacteroides fragilis* or *Bacteroides vulgatus.*

Preferably, the diversity index of the biomarker includes α-diversity index of the biomarker.

In the present application, the biomarkers *Escherichia coli, Clostridium ramosum, Eubacterium cylindroides, Roseburia hominis, Faecalibacterium prausnitzii, Bacteroides fragilis, Bacteroides vulgatus* and the α-diversity index of the biomarker are screened through the high-throughput sequencing in conjunction with a machine learning model as indicators for screening the fecal bacteria donor.

Preferably, a method for preparing the fecal bacteria liquid in step (2) includes the following steps:
(1") soaking the collected feces in sterile saline, and filtering to obtain a fecal filtrate;
(2") centrifuging the fecal filtrate, and mixing a precipitate with sterile saline to obtain the fecal bacteria liquid.

In the present application, the feces of the fecal bacteria donor are collected on-site using a sterile container, the collected feces are sent to a laboratory for information registration, fecal identification, weighing, evaluation and treatment within 1 h, and the preparation of the fecal bacteria liquid is completed in an anaerobic environment within 6 h of fecal donation.

Preferably, the sterile saline in step (1") has a temperature of 3-5 °C, which may be, for example, 3 °C, 4 °C or 5 °C.

Preferably, the filtration in step (1") is performed using a filter screen.

Preferably, the filter screen has an aperture of 0.25-2 mm, which may be, for example, 0.25 mm, 0.5 mm, 1.0 mm or 2.0 mm.

Preferably, the filtration in step (1") includes: removing the large particles by using 2.0 mm, 1.0 mm, 0.5 mm and 0.25 mm filter screens in sequence particles, and then filtering two to three times with the 0.25 mm filter screen to obtain a liquid phase as the fecal filtrate.

Preferably, the centrifugation in step (2") has a rotational speed of 1500-3000 r/min, which may be, for example, 1500 r/min, 1600 r/min, 1700 r/min, 1800 r/min, 1900 r/min, 2000 r/min, 2100 r/min, 2200 r/min, 2300 r/min, 2400 r/min, 2500 r/min, 2600 r/min, 2700 r/min, 2800 r/min, 2900 r/min or 3000 r/min.

Preferably, the centrifugation in step (2") is performed for 10-20 min, which may be, for example, 10 min, 11 min, 12 min, 13 min, 14 min, 15 min, 16 min, 17 min, 18 min, 19 min or 20 min.

Preferably, the lyophilizing protective agent in step (3) includes skim milk powder, trehalose, sucrose, vitamin C and sterile saline.

Preferably, the lyophilizing protective agent includes, in mass percentage, 10% to 20% skim milk powder, 10% to 15% trehalose, 1% to 10% sucrose, 1% to 5% vitamin C, and saline as balance.

In the present application, the lyophilizing protective agent can effectively prolong the survival time of flora and improve a colonization effect of flora.

Preferably, a volume ratio of the fecal bacteria liquid and the lyophilizing protective agent in step (3) is (2-5):1, which may be, for example, 2:1, 3:1, 4:1 or 5:1, preferably 3:1.

Preferably, conditions of the cooling and freezing in step (3) are as follows: lowering the temperature from room temperature to 3-6 °C for 10-20s, lowering the temperature from 3-6 °C to -30-(-50) °C at 1-2 °C/min, and lowering the temperature from -30-(-50) °C to -75-(-80) °C at 4-5 °C/min.

Preferably, the cooling and freezing in step (3) is performed for 12-24 h, which may be, for example, 12 h, 13 h, 14 h, 15 h, 16 h, 17 h, 18 h, 19 h, 20 h, 21 h, 22 h, 23 h or 24 h.

Preferably, the vacuum drying in step (3) has a vacuum degree of 5-15 pa, which may be, for example, 5 pa, 6 pa, 7 pa, 8 pa, 9 pa, 10 pa, 11 pa, 12 pa, 13 pa, 14 pa or 15 pa.

Preferably, the vacuum drying in step (3) is performed at -50-(-60) °C, which may be, for example, -50 °C, -51 °C, -52 °C, -53 °C, -54 °C, -55 °C, -56 °C, -57 °C, -58 °C, -59 °C or -60 °C.

Preferably, the vacuum drying in step (3) is performed for 24-48 h, which may be, for example, 24 h, 30 h, 36 h, 42 h or 48 h.

Preferably, the capsule shell in step (3) includes an enteric capsule shell.

In the present application, the enteric capsule shell can effectively resist the decomposition of gastric juice, release an effective flora in the capsule only under a condition of intestinal pH value, effectively protect the flora to reach the intestinal tract, prevent the premature release of the flora, reduce the activity loss of the flora, prolong the release time of the flora and achieve a specific colonization effect, which is conducive to achieving oral administration.

Preferably, the whole bacteria capsule is stored at -75-(-80) °C.

As a preferred technical solution, the present application provides a preparation method of a whole bacteria capsule. The preparation method includes the following steps:
(1) performing the second-generation sequencing on the DNA and/or RNA extracted from the fecal bacteria donor to obtain the original sequencing data; after removing the host gene from the original sequencing data, comparing the sequencing data with a National Center for Biotechnology Information (NCBI) microbial database for bacterial strains identification and abundance detection; comparing the sequencing data with a Kyoto Encyclopedia of Genes and Genomes (KEGG) pathogenic bacteria database to confirm no pathogenic bacteria in the fecal bacteria donor; and comparing the sequencing data with the intestinal flora of the fecal bacteria receptor and screening to obtain the fecal bacteria donor complementary to the fecal bacteria receptor;
(2) collecting the feces of the fecal bacteria donor screened in step (1), soaking the feces in the sterile saline (3-5 °C), and removing the large particles by using the 2.0 mm, 1.0 mm, 0.5 mm and 0.25 mm filter screens in sequence, and then filtering two to three times with the 0.25 mm filter screen to obtain the liquid phase as the fecal filtrate; and centrifuging the fecal filtrate for 10-20 min at 1500-3000 r/min and mixing the precipitate with the sterile saline to obtain the fecal bacteria liquid; and
(3) mixing the fecal bacteria liquid prepared in step (2) with the lyophilizing protective agent at a volume ratio of (2-5):1, wherein the lyophilizing protective agent comprises, in mass percentage, 10% to 20% skim milk powder, 10% to 15% trehalose, 1% to 10% sucrose, 1% to 5% vitamin C and the saline as the balance, and then lowering the temperature from room temperature to 3-6 °C within 10-20s, further lowering the temperature from 3-6 °C to -30-(-50) °C at a cooling rate of 1-2 °C/min and lowering the temperature from -30-(-50) °C to -75-(-80) °C at a cooling rate of 4-5 °C/min, and after the cooling and freezing, vacuum drying the mixture for 24-48 h under the conditions of a vacuum degree of 5-15 pa and -50-(-60) °C to obtain the lyophilized powder of fecal bacteria which is loaded into the enteric capsule shell to obtain the whole bacteria capsule which is stored at -75-(-80) °C.As a preferred technical solution, the present application provides a preparation method of a whole bacteria capsule. The preparation method includes the following steps:
   (1) performing the second-generation sequencing on the DNA and/or RNA extracted from the fecal bacteria donor to obtain the original sequencing data; and after removing the host gene from the original sequencing data, comparing the sequencing data with the NCBI microbial database and screening a fecal bacteria donor expressing any one or a combination of at least two of *Escherichia coli, Clostridium ramosum, Eubacterium cylindroides, Roseburia hominis, Faecalibacterium prausnitzii, Bacteroides fragilis* or *Bacteroides vulgatus;*
   (2) collecting the feces of the fecal bacteria donor screened in step (1), soaking the feces in the sterile saline (3-5 °C), and removing the large particles by using the 2.0 mm, 1.0 mm, 0.5 mm and 0.25 mm filter screens in sequence, and then filtering two to three times with the 0.25 mm filter screen to obtain the liquid phase as the fecal filtrate; and centrifuging the fecal filtrate for 10-20 min at 1500-3000 r/min and mixing the precipitate with the sterile saline to obtain the fecal bacteria liquid; and
   (3) mixing the fecal bacteria liquid prepared in step (2) with the lyophilizing protective agent at a volume ratio of (2-5):1, wherein the lyophilizing protective agent comprises, in mass percentage, 10% to 20% skim milk powder, 10% to 15% trehalose, 1% to 10% sucrose, 1% to 5% vitamin C and the saline as the balance, and then lowering the temperature from room temperature to 3-6 °C within 10-20s, further lowering the temperature from 3-6 °C to -30-(-50) °C at a cooling rate of 1-2 °C/min and lowering the temperature from -30-(-50) °C to -75-(-80) °C at a cooling rate of 4-5 °C/min, and after the cooling and freezing, vacuum drying the mixture for 24-48 h under the conditions of a vacuum degree of 5-15 pa and -50-(-60) °C to obtain the lyophilized powder of fecal bacteria which is loaded into the enteric capsule shell to obtain the whole bacteria capsule which is stored at -75-(-80) °C.

In a second aspect, the present application provides a whole bacteria capsule. The whole bacteria capsule is prepared through the preparation method according to the first aspect.

In a third aspect, the present application provides a pharmaceutical composition. The pharmaceutical composition includes the whole bacteria capsule according to the second aspect.

Preferably, the pharmaceutical composition further includes a PD-1 inhibitor.

Preferably, the pharmaceutical composition further includes any one or a combination of at least two of a pharmaceutically acceptable carrier, diluent or excipient.

In a fourth aspect, the present application provides a use of the whole bacteria capsule according to the second aspect and/or the pharmaceutical composition according to the third aspect for preparing a drug for treating a disease.

Preferably, the disease includes any one or a combination of at least two of inflammatory bowel disease, intestinal polyp, adenoma, bowel cancer, hepatic encephalopathy, graft-versus-host disease or *Clostridium difficile* infection.

Compared with the existing art, the present application has the beneficial effects below.
(1) In the present application, the fecal bacteria donor is screened through the high-throughput sequencing, and the sequencing data of the fecal bacteria donor is compared with the public database and compared with the intestinal flora of the fecal bacteria receptor (the patient) so that the fecal bacteria donors for different fecal bacteria receptors can be quickly screened and an identification level reaches the strain level. Meanwhile, the high-throughput sequencing can also identify the pathogenic bacteria, excludes the fecal bacteria donor with a potential risk and performs the quality monitoring.
(2) The fecal bacterial flora screened in the present application is mixed with the lyophilizing protective agent and subjected to lyophilizing treatment, effectively prolonging the survival time of flora and improving the colonization effect of flora.
(3) The whole bacteria capsule in the present application achieves the "accurate match" for the fecal bacteria receptor and has a significant pharmaceutical effect. After three doses, patients show a significant decrease in an abdominal pain score, the number of defecation per day and the number of food types of second-grade food intolerance, and a significant increase in the abundance of Firmicutes, which tends to be consistent with the healthy donor.

### BRIEF DESCRIPTION OF DRAWINGS

FIG. 1 is a flowchart for screening a fecal bacteria donor based on high-throughput sequencing.
FIG. 2 is a diagram illustrating a comparison between scores of irritable bowel syndrome scale before and after oral administration of a whole bacteria capsule in a patient with irritable bowel syndrome.
FIG. 3 is a diagram illustrating a differential flora before and after oral administration of a whole bacteria capsule in a patient with irritable bowel syndrome and a relationship with a donor.

### DETAILED DESCRIPTION

To further elaborate on the technical means adopted and effects achieved in the present application, the present application is described below in conjunction with examples and drawings. It is to be understood that the specific examples set forth below are intended to explain the present application and not to limit the present application.

Experiments without specific techniques or conditions specified in the examples are conducted according to techniques or conditions described in the literature in the art or a product specification. The reagents or instruments used herein without manufacturers specified are conventional products commercially available from proper channels.

### Example 1 Determination criteria of a healthy donor

Feces were derived from a healthy donor. An inclusion criterion of the healthy donor is as follows:
(1) healthy, unmarried men and women aged 18 to 23;
(2) living a regular life and having no adverse hobbies;
(3) no history of antibiotic use within three months;
(4) no gastrointestinal tract disease;
(5) negative serology and fecal infectious pathogen examination and culture; and
(6) good types and diversity of an intestinal flora: types of a probiotic and the diversity of a flora are determined to be good after high-throughput detection of a 16S rDNA gene of the intestinal flora.

An exclusion criterion of the healthy donor is as follows:
(1) history of drug administration: those who have used an antibiotic, a laxative or a weight-loss drug within three months or those who are taking an immunosuppressive drug or a chemotherapy drug;
(2) history of virus exposure: those who have been or have recently been exposed to HIV virus, hepatitis A virus, hepatitis B virus or hepatitis C virus (HCV);
(3) history of disease: those with psychiatric disorder, malignant obesity, constipation, diabetes, inflammatory bowel disease, allergy, metabolic syndrome, hypoimmunity, autoimmune disease, intestine-related disorder, chronic fatigue syndrome, history of digestive system surgery, gastrointestinal malignant tumor or polyp, or atopic disease (such as eczema, asthma and gastrointestinal eosinophil-related disorder);
(4) virology and etiology: *Helicobacter pylori, Yersinia, Campylobacter, Shigella, Salmonella,* enteropathogenic *Escherichia coli,* rotavirus, adenovirus, astrovirus, Toxoplasma gondii and Giardia are detected in a fecal sample;
(5) human immunodeficiency virus (HIV), human T-lymphotropic virus (HTLV), hepatitis A virus (HAV), hepatitis B virus (HBV), hepatitis C virus (HCV), cytomegalovirus, Epstein-Barr virus (EBV), syphilis, Strongyloides, amoeba protozoa and the like are detected through serological detection; and
(6) a history of high-risk sexual behaviour, a history of drug use or illicit drug use, a recent history of imprisonment or a recent history of travel to an epidemic area.

People who met all the conditions of the above donor inclusion criterion and did not have any one of the donor exclusion criterion were determined as the healthy donor.

### Example 2 Screening of a fecal bacteria donor based on high-throughput sequencing

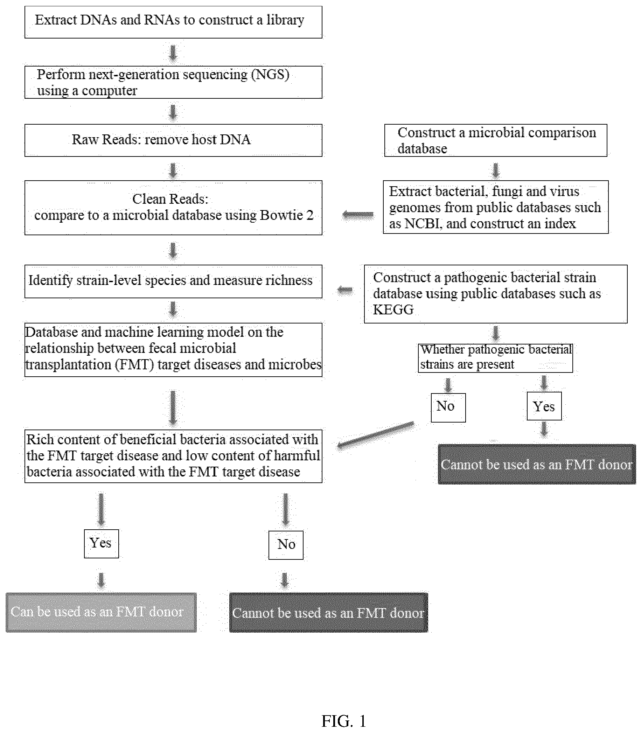

The people determined as the healthy donor according to Example 1 were used as a fecal bacteria donor and further screened based on a high-throughput sequencing technology. A flowchart is shown in FIG. 1, and steps are described below.

DNA was extracted from feces of the fecal bacteria donor, and a library was constructed for second-generation sequencing to obtain original sequencing data. After a host gene was removed from the original sequencing data, the sequencing data was compared with an NCBI microbial database (a bacterial genome, a fungal genome and a viral genome) for bacterial strains identification and abundance detection; the sequencing data was compared with a KEGG pathogenic bacteria database to confirm no pathogenic bacteria in the fecal bacteria donor; and the sequencing data was compared with an intestinal flora of a fecal bacteria receptor and screened to obtain a fecal bacteria donor complementary to the fecal bacteria receptor.

In this example, the screened fecal bacteria donor expressed biomarkers *Escherichia coli, Clostridium ramosum, Eubacterium cylindroides, Roseburia hominis, Faecalibacterium prausnitzii, Bacteroides fragilis* and *Bacteroides vulgatus,* and α diversity of the biomarkers was 163804.8. Biomarkers positively correlating to health were *Clostridium ramosum, Eubacterium cylindroides, Roseburia hominis, Faecalibacterium prausnitzii* and *Bacteroides vulgatus,* and biomarkers negatively correlating to health were *Escherichia coli* and *Bacteroides fragilis.*

In healthy people, a box plot analysis was performed on each microorganism positively correlating to health to obtain a median value for the each microorganism. In patients, the box plot analysis was performed on each microorganism negatively correlating to health to obtain a median value for the each microorganism. The median value for the each microorganism was used as a standard value for the donor screening.

A screening criterion of the fecal bacteria donor is as follows: a value of each microorganism positively correlating to health is greater than the standard value of the each microorganism, and a value of each microorganism negatively correlating to health is less than the standard value of the each microorganism.

### Example 3 Preparation of a fecal bacteria liquid

The screened feces of the fecal bacteria donor screened in Example 2 that was specific for the fecal bacteria receptor were collected on-site and sent to a laboratory for information registration, fecal identification, weighing, evaluation and treatment within 1 h to prepare a fecal bacteria liquid in an anaerobic environment. Steps are described below.
(1) The collected feces were soaked in sterile saline (5 °C), and large particles were removed by using 2.0 mm, 1.0 mm, 0.5 mm and 0.25 mm filter screens in sequence, and then filtered three times with the 0.25 mm filter screen to obtain a liquid phase as a fecal filtrate.
(2) The fecal filtrate was centrifuged for 10 min at 3000 r/min, and a precipitate was mixed with sterile saline to obtain the fecal bacteria liquid.

### Example 4 Preparation of a fecal bacteria liquid

The screened feces of the fecal bacteria donor screened in Example 2 that was specific for the fecal bacteria receptor were collected on-site and sent to a laboratory for information registration, fecal identification, weighing, evaluation and treatment within 1 h to prepare a fecal bacteria liquid in an anaerobic environment. Steps are described below.
(1) The collected feces were soaked in sterile saline (3 °C), and large particles were removed by using 2.0 mm, 1.0 mm, 0.5 mm and 0.25 mm filter screens, and then filtered two times with the 0.25 mm filter screen to obtain a liquid phase as a fecal filtrate.
(2) The fecal filtrate was centrifuged for 20 min at 1500 r/min, and a precipitate was mixed with sterile saline to obtain the fecal bacteria liquid.

### Example 5 Preparation of a whole bacteria capsule

The fecal bacteria liquid prepared in Example 3 was mixed with a lyophilizing protective agent (15% skim milk powder, 15% trehalose, 5% sucrose, 5% vitamin C and saline as balance) according to 3:1 (v/v). The temperature was lowered from room temperature to 4 °C within 10s, further lowered from 4 °C to -40 °C at a cooling rate of 2 °C/min and lowered from -40 °C to -80 °C at a cooling rate of 5 °C/min. After cooling and freezing, the mixture was vacuum dried for 48 h under conditions of a vacuum degree of 10 pa and -50 °C to obtain lyophilized powder of fecal bacteria which was loaded into an enteric capsule shell to obtain the whole bacteria capsule which was stored at -80 °C.

### Example 6 Preparation of a whole bacteria capsule

The fecal bacteria liquid prepared in Example 4 was mixed with a lyophilizing protective agent (10% skim milk powder, 15% trehalose, 1% sucrose, 5% vitamin C and saline as balance). The temperature was lowered from room temperature to 6 °C within 10s, further lowered from 6 °C to -50 °C at a cooling rate of 2 °C/min and lowered from -50 °C to -75 °C at a cooling rate of 5 °C/min. After cooling and freezing, the mixture was vacuum dried for 48 h under conditions of a vacuum degree of 5 pa and -50 °C to obtain lyophilized powder of fecal bacteria which was loaded into an enteric capsule shell to obtain the whole bacteria capsule which was stored at -80 °C.

### Example 7 Preparation of a whole bacteria capsule

The fecal bacteria liquid prepared in Example 4 was mixed with a lyophilizing protective agent (20% skim milk powder, 10% trehalose, 10% sucrose, 1% vitamin C and saline as balance). The temperature was lowered from room temperature to 3 °C within 20s, further lowered from 3 °C to -30 °C at a cooling rate of 1 °C/min and lowered from -30 °C to -80 °C at a cooling rate of 4 °C/min. After cooling and freezing, the mixture was vacuum dried for 24 h under conditions of a vacuum degree of 15 pa and -60 °C to obtain lyophilized powder of fecal bacteria which was loaded into an enteric capsule shell to obtain the whole bacteria capsule which was stored at -75 °C.

### Example 8 Therapeutic effect of the whole bacteria capsule

Patients with irritable bowel syndrome were administrated three doses of the whole bacteria capsule prepared in Example 5: once a week for three weeks.

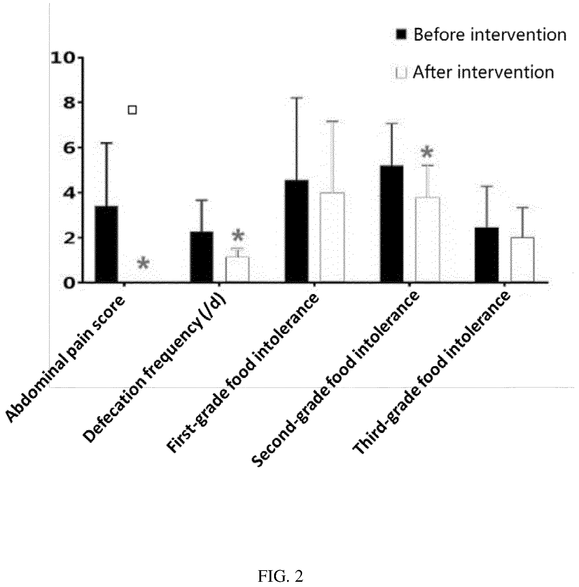

As shown in FIG. 2, there was a significant decrease (p < 0.01) in the abdominal pain score, the number of defecation per day and the number of food types of second-grade food intolerance, as well as a corresponding decrease in the number of food types of first-grade food intolerance and the number of food types of third-grade food intolerance.

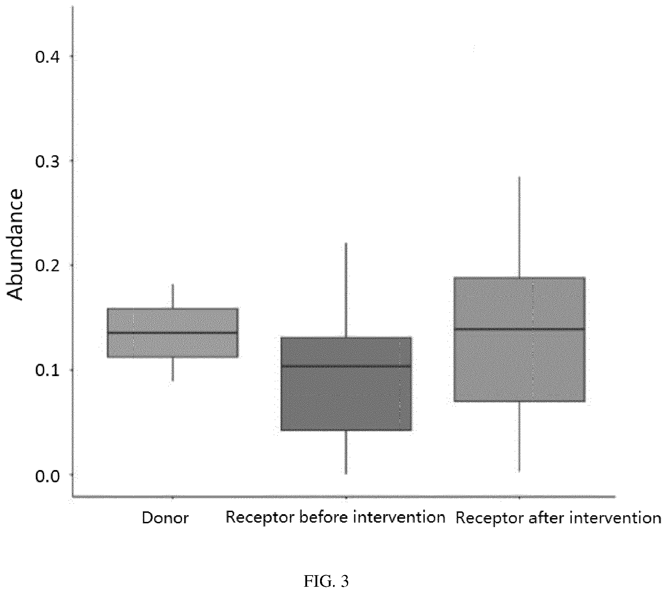

As shown in FIG. 3, 16S rRNA detection was performed on fecal samples from patients. Samples showed a significant increase (p < 0.01) in the abundance of Firmicutes at a family classification level, which tends to be consistent with the healthy donor.

To conclude, the fecal bacteria donor is screened through the high-throughput sequencing, accurately screening the fecal bacteria donors that are specific for different fecal bacteria receptors, and the prepared whole bacteria capsule is "matched accurately" with a significant pharmaceutical effect and high safety.

The applicant has stated that although the detailed method of the present application is described through the examples described above, the present application is not limited to the detailed method described above, which means that the implementation of the present application does not necessarily depend on the detailed method described above. It should be apparent to those skilled in the art that any improvements made to the present application, equivalent replacements of raw materials of the product of the present application, additions of adjuvant ingredients, selections of specific manners, etc., all fall within the protection scope and the disclosure scope of the present application.

## Claims

1. A preparation method of a whole bacteria capsule, comprising the following steps:
(1) screening a fecal bacteria donor through high-throughput sequencing;
(2) collecting feces of the fecal bacteria donor screened in step (1) to prepare a fecal bacteria liquid; and
(3) mixing the fecal bacteria liquid prepared in step (2) with a lyophilizing protective agent and cooling, freezing and vacuum drying the mixture to obtain lyophilized powder of fecal bacteria which is loaded into a capsule shell to obtain the whole bacteria capsule.

2. The preparation method according to claim 1, wherein a method for screening the fecal bacteria donor through the high-throughput sequencing in step (1) comprises the following steps:
(1') performing second-generation sequencing on DNA and/or RNA extracted from the fecal bacteria donor to obtain original sequencing data;
(2') after removing a host gene from the original sequencing data, comparing the sequencing data with a microbial database for bacterial strains identification and abundance detection;
(3') comparing the sequencing data with a pathogenic bacteria database to confirm no pathogenic bacteria in the fecal bacteria donor; and
(4') comparing the sequencing data with an intestinal flora of a fecal bacteria receptor and screening to obtain a fecal bacteria donor complementary to the fecal bacteria receptor.

3. The preparation method according to claim 1 or 2, wherein the microbial database in step (2') comprises any one or a combination of at least two of a bacterial genome, a fungal genome or a viral genome derived from a public database.

4. The preparation method according to any one of claims 1 to 3, wherein the pathogenic bacteria database in step (3') comprises a pathogenic bacterial genome derived from the public database.

5. The preparation method according to any one of claims 1 to 4, wherein screening the fecal bacteria donor through the high-throughput sequencing in step (1) is performed according to a biomarker expressed by the fecal bacteria donor and a diversity index of the biomarker.

6. The preparation method according to any one of claims 1 to 5, wherein the biomarker comprises any one or a combination of at least two of *Escherichia coli, Clostridium ramosum, Eubacterium cylindroides, Roseburia hominis, Faecalibacterium prausnitzii, Bacteroides fragilis* or *Bacteroides vulgatus.*

7. The preparation method according to any one of claims 1 to 6, wherein the diversity index of the biomarker comprisesα-diversity index of the biomarker.

8. The preparation method according to any one of claims 1 to 7, wherein a method for preparing the fecal bacteria liquid in step (2) comprises the following steps:
(1") soaking the collected feces in sterile saline and filtering to obtain a fecal filtrate;
(2") centrifuging the fecal filtrate and mixing a precipitate with sterile saline to obtain the fecal bacteria liquid;
preferably, the sterile saline in step (1") has a temperature of 3-5 °C;
preferably, the filtration in step (1") is performed using a filter screen;
preferably, the filter screen has an aperture of 0.25-2 mm;
preferably, the filtration in step (1") comprises: removing the large particles by using 2.0 mm, 1.0 mm, 0.5 mm and 0.25 mm filter screens in sequence and then filtering two to three times with the 0.25 mm filter screen to obtain a liquid phase as the fecal filtrate;
preferably, the centrifugation in step (2") has a rotational speed of 1500-3000 r/min;
preferably, the centrifugation in step (2") is performed for 10-20 min.

9. The preparation method according to any one of claims 1 to 8, wherein the lyophilizing protective agent in step (3) comprises skim milk powder, trehalose, sucrose, vitamin C and sterile saline;
preferably, the lyophilizing protective agent comprises, in mass percentage, 10% to 20% skim milk powder, 10% to 15% trehalose, 1% to 10% sucrose, 1% to 5% vitamin C and saline as balance;
preferably, a volume ratio of the fecal bacteria liquid and the lyophilizing protective agent in step (3) is (2-5):1;
preferably, conditions of the cooling and freezing in step (3) are as follows: lowering the temperature from room temperature to 3-6 °C for 10-20s, lowering the temperature from 3-6 °C to -30-(-50) °C at 1-2 °C/min and lowering the temperature from -30-(-50) °C to -75-(-80) °C at 4-5 °C/min;
preferably, the cooling and freezing in step (3) is performed for 12-24 h;
preferably, the vacuum drying in step (3) has a vacuum degree of 5-15 pa;
preferably, the vacuum drying in step (3) is performed at -50-(-60) °C;
preferably, the vacuum drying in step (3) is performed for 24-48 h;
preferably, the capsule shell in step (3) comprises an enteric capsule shell; and
preferably, the whole bacteria capsule is stored at -75-(-80) °C.

10. The preparation method according to any one of claims 1 to 9, comprising the following steps:
(1) performing the second-generation sequencing on the DNA and/or RNA extracted from the fecal bacteria donor to obtain the original sequencing data; after removing the host gene from the original sequencing data, comparing the sequencing data with a National Center for Biotechnology Information (NCBI) microbial database for bacterial strains identification and abundance detection; comparing the sequencing data with a Kyoto Encyclopedia of Genes and Genomes (KEGG) pathogenic bacteria database to confirm no pathogenic bacteria in the fecal bacteria donor; and comparing the sequencing data with the intestinal flora of the fecal bacteria receptor and screening to obtain the fecal bacteria donor complementary to the fecal bacteria receptor;
(2) collecting the feces of the fecal bacteria donor screened in step (1), soaking the feces in the sterile saline (3-5 °C), and removing the large particles by using the 2.0 mm, 1.0 mm, 0.5 mm and 0.25 mm filter screens in sequence, and then filtering two to three times with the 0.25 mm filter screen to obtain the liquid phase as the fecal filtrate; and centrifuging the fecal filtrate for 10-20 min at 1500-3000 r/min and mixing the precipitate with the sterile saline to obtain the fecal bacteria liquid; and
(3) mixing the fecal bacteria liquid prepared in step (2) with the lyophilizing protective agent at a volume ratio of (2-5):1, wherein the lyophilizing protective agent comprises, in mass percentage, 10% to 20% skim milk powder, 10% to 15% trehalose, 1% to 10% sucrose, 1% to 5% vitamin C and the saline as the balance, and then lowering the temperature from room temperature to 3-6 °C within 10-20s, further lowering the temperature from 3-6 °C to -30-(-50) °C at a cooling rate of 1-2 °C/min and lowering the temperature from -30-(-50) °C to -75-(-80) °C at a cooling rate of 4-5 °C/min, and after the cooling and freezing, vacuum drying the mixture for 24-48 h under the conditions of a vacuum degree of 5-15 pa and -50-(-60) °C to obtain the lyophilized powder of fecal bacteria which is loaded into the enteric capsule shell to obtain the whole bacteria capsule which is stored at -75-(-80) °C.

11. The preparation method according to any one of claims 1 to 10, comprising the following steps:
(1) performing the second-generation sequencing on the DNA and/or RNA extracted from the fecal bacteria donor to obtain the original sequencing data; and after removing the host gene from the original sequencing data, comparing the sequencing data with the NCBI microbial database and screening a fecal bacteria donor expressing any one or a combination of at least two of *Escherichia coli, Clostridium ramosum, Eubacterium cylindroides, Roseburia hominis, Faecalibacterium prausnitzii, Bacteroides fragilis* or *Bacteroides vulgatus;*
(2) collecting the feces of the fecal bacteria donor screened in step (1), soaking the feces in the sterile saline (3-5 °C), and removing the large particles by using the 2.0 mm, 1.0 mm, 0.5 mm and 0.25 mm filter screens in sequence, and then filtering two to three times with the 0.25 mm filter screen to obtain the liquid phase as the fecal filtrate; and centrifuging the fecal filtrate for 10-20 min at 1500-3000 r/min and mixing the precipitate with the sterile saline to obtain the fecal bacteria liquid; and
(3) mixing the fecal bacteria liquid prepared in step (2) with the lyophilizing protective agent at a volume ratio of (2-5): 1, wherein the lyophilizing protective agent comprises, in mass percentage, 10% to 20% skim milk powder, 10% to 15% trehalose, 1% to 10% sucrose, 1% to 5% vitamin C and the saline as the balance, and then lowering the temperature from room temperature to 3-6 °C within 10-20s, further lowering the temperature from 3-6 °C to -30-(-50) °C at a cooling rate of 1-2 °C/min and lowering the temperature from -30-(-50) °C to -75-(-80) °C at a cooling rate of 4-5 °C/min, and after the cooling and freezing, vacuum drying the mixture for 24-48 h under the conditions of a vacuum degree of 5-15 pa and -50-(-60) °C to obtain the lyophilized powder of fecal bacteria which is loaded into the enteric capsule shell to obtain the whole bacteria capsule which is stored at -75-(-80) °C.

12. A whole bacteria capsule which is prepared through the preparation method according to any one of claims 1 to 11.

13. A pharmaceutical composition, comprising the whole bacteria capsule according to claim 12;
preferably, the pharmaceutical composition further comprises a PD-1 inhibitor;
preferably, the pharmaceutical composition further comprises any one or a combination of at least two of a pharmaceutically acceptable carrier, diluent or excipient.

14. A use of the whole bacteria capsule according to claim 12 and/or the pharmaceutical composition according to claim 13 for preparing a drug for treating a disease;
preferably, the disease comprises any one or a combination of at least two of inflammatory bowel disease, intestinal polyp, adenoma, bowel cancer, hepatic encephalopathy, graft-versus-host disease or *Clostridium difficile* infection.
